# EUROPEAN PATENT APPLICATION

(11) **EP 2 915 506 A1**
(43) Date of publication of application: **09.09.2015**
(21) Application number: 13850659.7
(22) Date of filing: 24.10.2013
(51) Int. Cl.: A61F 2/36

(54) **ARTIFICIAL HIP JOINT**

(30) Priority: 31.10.2012 JP 2012240097
(71) Applicant: Kyocera Medical Corporation, Osaka-shi, Osaka 532-0003 (JP)
(72) Inventor: WAKIYAMA, Miyo, Osaka-shi Osaka 532-0003 (JP); SHIMOZONO, Takayoshi, Osaka-shi Osaka 532-0003 (JP)
(74) Representative: Viering, Jentschura & Partner Patent- und Rechtsanwälte
(86) International application number: PCT/JP2013/078863
(87) International publication number: WO 2014/069333

(57) **Abstract**

The invention relates to an artificial hip joint in which a length of fit can be ensured without increasing the overall height of the head, and stress generated in the head can be alleviated by avoiding concentration of stress. An artificial hip joint includes a stem (6) having a neck portion (5) which has a truncated conical outer circumferential surface (4) decreased in diameter toward a tip (3) thereof; a head (8) provided with a concavity (7) in which the neck portion (5) is inserted, an inner surface of the concavity (7) of the head including a truncated conical inner circumferential surface (10) decreased in diameter toward an insertion direction of inserting the neck portion (5); and a sleeve (2A) inserted between the outer circumferential surface (4) of the neck portion (5) inserted into the concavity (7) and the inner circumferential surface (10) of the head (8). The sleeve (2A) has an inner circumferential portion (13) which makes contact with the outer circumferential surface (4) of the neck portion (5), and an outer circumferential portion (16) which makes contact with the inner circumferential surface (10) of the head (8), a starting end of the outer circumferential portion being located at a position retracting from an opening portion (14) of the concavity (7) in the insertion direction A.

## Description

### Technical Field

The present invention relates to an artificial hip joint in which a neck portion of a stem fits a tapered concavity of a head so as to join the head and the stem together.

### Background Art

Conventionally, an artificial hip joint is made of metal such as stainless steel, a cobalt-chromium alloy, or a titanium alloy, and has a stem inserted into and fixed to a thighbone, and a ceramic head, wherein the head and the stem are fixed in an integral manner or by performing taper-fitting, and a polyethylene cup is fixed onto an acetabular cartridge side in which the head is received.

The heads mostly have a taper-fitting structure so as to be fixed to a neck portion formed at a tip portion of a metal stem. The head is configured to be adjustable in length of fit (also referred to as "a neck offset") when being attached to the stem, by changing the depth or the inner diameter of a concavity which is also called a tapered hole.

In consideration of combination with a polyethylene cup provided on the acetabular cartridge side, a head made of ceramics such as alumina or zirconia which is a low frictional and low wear-out material is clinically used. However, the ceramic head has a problem in that damage is likely to occur due to incompatibility between the neck portion at the tip portion of the metallic stem to be joined and the concavity formed in the head.

Incidentally, it is said that a load equal to or greater than five times one's weight at most is applied to a femur head. For example, in a case of a person weighing 80 kg, a maximum load of approximately 400 kg is repeatedly applied thereto. In this manner, since a great force is constantly applied to a hip joint of a human body for a long period of time, great strength is required for the artificial hip joint.

In addition, a high safety factor is required for the artificial hip joint from the viewpoint of durability on a long-term basis. However, in practice, when the neck portion of the stem is inserted into the concavity formed in the ceramic head, stress distribution in a concavity section of the head becomes irregular due to even incompatibility caused by a slight scratch in the neck portion, leading to a problem of inducing a rupture in the head caused by local stress concentration.

In order to solve such problems, in the artificial hip joint in which the neck portion of the stem is inserted into and fixed to the concavity formed in the ceramic head, there is utilized a technology of causing a conical sleeve to be inserted between an inner circumferential surface of the concavity and an outer circumferential surface of the neck portion of the stem.

In such a conventional technology, the length of fit, that is, the neck offset of the neck portion with respect to the head is easily changed by adjusting the thickness of the sleeve, and thus, without preparing various types of the ceramic heads, it is possible to cope with a plurality of different lengths of fit even with one type of the head (for example, refer to Patent Literature 1, Patent Literature 2, and Non-Patent Literature 1).

### Citation List

### Patent Literature

Patent Literature 1: Japanese Unexamined Patent Publication JP-A 3-47253 (1991)
Patent Literature 2: Japanese Unexamined Patent Publication JP-A 2002-330983

### Non-Patent Literature

Non-Patent Literature 1: "NEWS RELEASE, Information about New Technology and New Products: New Generation Ceramic Head and Liner Containing Vitamin E for Artificial Hip Joint Tied Up with Latest Technology Introduced in Japan" [online], May 15, 2012, Biomet Japan Inc., [searched on October 29, 2012], Internet (URL: https://www.biomet.co.jp/information/img/Biomet_Release051 5.pdf)

### Summary of Invention

### Technical Problem

In the above-described conventional technology, fracture strength of a ceramic head is enhanced by varying the thickness of a sleeve and causing a neck portion to fit a concavity at a position deeper than an opening portion of the concavity in an insertion direction of the neck portion. However, as the length of fit of the neck portion and the sleeve becomes short, there is a high possibility that the neck portion fits obliquely. In contrast, if the overall height of the head is increased in order to ensure the length of fit, it is difficult to ensure a sufficient thickness at a stress concentration portion generated in the vicinity of the opening portion of the concavity, thereby leading to a problem in that necessary strength cannot be achieved.

An object of the invention is to provide an artificial hip joint in which the length of fit can be ensured without increasing the overall height of the head, and stress generated in the head can be reduced by mitigating concentration of stress.

### Solution to Problem

The invention provides an artificial hip joint including:
a stem having a neck portion which has a truncated conical outer circumferential surface decreased in diameter toward a tip thereof,
a head provided with a concavity in which the neck portion is inserted, an inner surface of the concavity of the head including a truncated conical inner circumferential surface decreased in diameter toward an insertion direction of inserting the neck portion, and
a sleeve being inserted between an outer circumferential surface of the neck portion inserted into the concavity and an inner circumferential surface of the head,
the sleeve having an inner circumferential portion which makes contact with the outer circumferential surface of the neck portion, and an outer circumferential portion which makes contact with the inner circumferential surface of the head in a region farther away than an opening portion of the concavity in the insertion direction.

Furthermore, the invention provides an artificial hip joint including:
a stem having a neck portion which has a truncated conical outer circumferential surface decreased in diameter toward a tip thereof,
a head provided with a concavity in which the neck portion is inserted, an inner surface of the concavity of the head including a truncated conical inner circumferential surface decreased in diameter toward an insertion direction of inserting the neck portion, and
a sleeve inserted between an outer circumferential surface of the neck portion inserted into the concavity and an inner circumferential surface of the head,
the sleeve having an inner circumferential portion which makes contact with the outer circumferential surface of the neck portion, and an outer circumferential portion which makes contact with the inner circumferential surface of the head, the sleeve being provided with a slit which is formed between the inner circumferential portion and the outer circumferential portion so as to extend in the insertion direction from one end portion of the sleeve located on an opening portion side of the concavity.

Furthermore, the invention provides an artificial hip joint including:
a stem having a neck portion which has a truncated conical outer circumferential surface decreased in diameter toward a tip thereof,
a head provided with a concavity in which the neck portion is inserted, an inner surface of the concavity of the head including a truncated conical inner circumferential surface decreased in diameter toward an insertion direction of inserting the neck portion, and
a sleeve inserted between an outer circumferential surface of the neck portion inserted into the concavity and an inner circumferential surface of the head,
the sleeve having a multi-layer structure in which a plurality of sleeve sections are stacked.

Furthermore, the invention provides an artificial hip joint including:
a stem having a neck portion which has a truncated conical outer circumferential surface decreased in diameter toward a tip thereof,
a head provided with a concavity in which the neck portion is inserted, an inner surface of the concavity of the head including a truncated conical inner circumferential surface decreased in diameter toward an insertion direction of inserting the neck portion, and
a sleeve inserted between an outer circumferential surface of the neck portion inserted into the concavity and an inner circumferential surface of the head,
the sleeve having an inner circumferential portion which makes contact with the outer circumferential surface of the neck portion, an outer circumferential portion which makes contact with the inner circumferential surface of the concavity, and a flange portion which makes contact with a peripheral surface of an opening portion of the concavity.

In the invention, it is preferable that the sleeve is made of Ti or a Ti alloy.

### Advantageous Effects of Invention

According to the invention, an inner circumferential portion of a sleeve makes contact with an outer circumferential surface of a neck portion, and an outer circumferential portion of the sleeve makes contact with an inner circumferential surface of a head at a region farther away than an opening portion of a concavity in an insertion direction. Thus, a stress concentration portion generated in the head can be moved to a site having the cross-sectional area larger than the opening portion of the head. Accordingly, it is possible to provide an artificial hip joint in which stress generated in the head can be reduced by mitigating concentration of stress. Since the length of fit of the outer circumferential surface of the neck portion and the inner circumferential portion of the sleeve which is in contact therewith can achieve a sufficient length, it is possible to ensure fixing strength of the head with respect to the neck portion. Accordingly, it is possible to realize miniaturization of the head by lowering the overall height of the head to the extent that the minimal necessary sliding area of the head can be obtained. Since the inner circumferential portion of the sleeve becomes a guide when the head is mounted on the neck portion, it is possible to lower the possibility that the head and the neck portion fit obliquely.

In addition, according to the invention, the inner circumferential portion of the sleeve makes contact with the outer circumferential surface of the neck portion, the outer circumferential portion of the sleeve makes contact with the inner circumferential surface of the head, and a slit extending in the insertion direction from one end portion of the sleeve is formed between the inner circumferential portion and the outer circumferential portion thereof. Accordingly, it is possible to mitigate concentration of stress by moving the stress concentration portion generated in the head to the site having the cross-sectional area larger than the opening portion of the head.

Furthermore, according to the invention, the sleeve is realized by a multi-layer structure in which a plurality of sleeve sections are stacked. Accordingly, sliding is generated at interfaces of the plurality of sleeve sections, and thus, it is possible to mitigate stress generated in the head.

Furthermore, according to the invention, the inner circumferential portion of the sleeve makes contact with the outer circumferential surface of the neck portion, the outer circumferential portion of the sleeve makes contact with the inner circumferential surface of the concavity, and a flange portion makes contact with a peripheral surface of the opening portion of the concavity. Accordingly, it is possible to mitigate stress by causing the concentration of stress generated in the vicinity of the opening portion of the head to disperse in a direction along the outer circumferential portion of the sleeve and a direction along the flange portion.

Furthermore, according to the invention, the sleeve is made of Ti or a Ti alloy suitable for corrosion resistance and malleability. Thus, it is possible to improve strength of a caput of the artificial hip joint, and simultaneously, it is possible to improve a fitting force of the caput and the neck.

### Brief Description of Drawings

The objects, characteristics, and advantages of the invention will become clearer through the following descriptions and drawings in detail.
FIG. 1 is a cross-sectional view illustrating an artificial hip joint 1A according to Embodiment 1 of the invention;
FIG. 2 is a perspective view of a sleeve 2A which is used in the artificial hip joint 1A illustrated in FIG. 1;
FIG. 3 is a diagram illustrating a stress analysis model of an artificial hip joint;
FIG. 4 is a stress distribution diagram showing an analysis result of Example 1 simulating the artificial hip joint 1A illustrated in FIG. 1;
FIG. 5 is a stress distribution diagram showing an analysis result of Comparison Example 1;
FIG. 6 is a stress distribution diagram showing an analysis result of Comparison Example 2;
FIG. 7 is a cross-sectional view illustrating an artificial hip joint 1B according to Embodiment 2 of the invention;
FIG. 8 is a perspective view of a sleeve 2B which is used in the artificial hip joint 1B illustrated in FIG. 7;
FIG. 9 is a stress distribution diagram showing an analysis result of Example 2 simulating the artificial hip joint 1B illustrated in FIG. 7;
FIG. 10 is a cross-sectional view illustrating an artificial hip joint 1C according to Embodiment 3 of the invention;
FIG. 11 is a perspective view of a sleeve 2C which is used in the artificial hip joint 1C illustrated in FIG. 10;
FIG. 12 is an exploded perspective view of the sleeve 2C which is used in the artificial hip joint 1C illustrated in FIG. 10;
FIG. 13 is a stress distribution diagram showing a stress analysis result of Example 3 simulating the artificial hip joint 1C illustrated in FIG. 10;
FIG. 14 is a cross-sectional view illustrating an artificial hip joint 1D according to Embodiment 4 of the invention;
FIG. 15 is a perspective view of a sleeve 2D which is used in the artificial hip joint 1D illustrated in FIG. 14 ; and
FIG. 16 is a stress distribution diagram showing a stress analysis result of Example 4 simulating the artificial hip joint 1D illustrated in FIG. 14.

### Description of Embodiments

Hereinafter, suitable embodiments of the invention will be described in detail with reference to the drawings.

### (Embodiment 1)

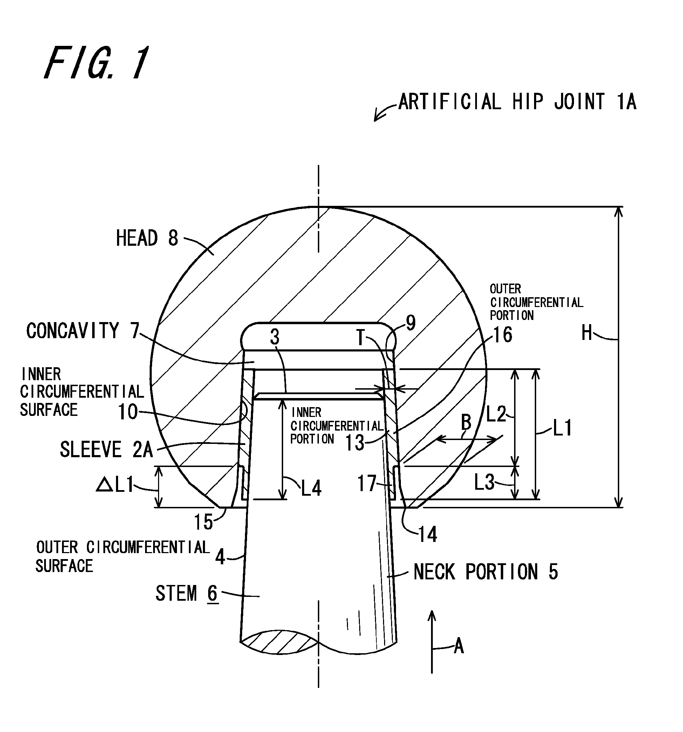

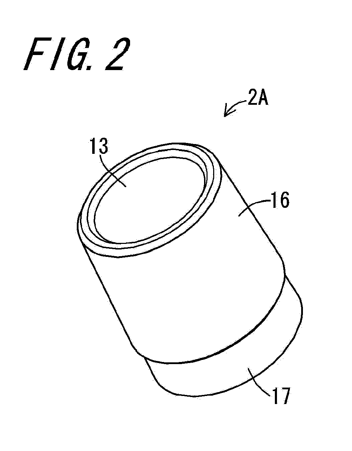

FIG. 1 is a cross-sectional view illustrating an artificial hip joint 1A according to Embodiment 1 of the invention, and FIG. 2 is a perspective view of a sleeve 2A which is used in the artificial hip joint 1A illustrated in FIG. 1. The artificial hip joint 1A of the present embodiment includes a stem 6, a head 8, and the sleeve 2A. The stem 6 has a neck portion 5 which has a truncated conical outer circumferential surface 4 decreased in diameter toward a tip 3 thereof. The head 8 is provided with a concavity 7 in which the neck portion 5 is inserted, and the head 8 has an inner surface 9 defining the concavity 7 which inner surface includes a truncated conical inner circumferential surface 10 decreased in diameter toward an insertion direction A of inserting the neck portion 5. The sleeve 2A is inserted between the outer circumferential surface 4 of the neck portion 5 inserted into the concavity 7, and the inner circumferential surface 10 of the head 8.

The stem 6 is made of metal such as stainless steel, a cobalt-chromium alloy, or a titanium alloy. An end portion of the stem 6 which is opposite to a side inserted into a thighbone is provided with the neck portion 5 on which the head 8 is mounted. As the head 8, a head made of ceramics such as alumina or zirconia which is a low frictional and low wear material is employed.

The sleeve 2A is made of titanium (Ti) or a titanium alloy, and the sleeve 2A is an annular member having a substantially truncated conical outer shape. When using the sleeve 2A, the sleeve 2A may be mounted inside the concavity 7 of the head 8 in advance. There are multiple types of standards for a taper shape of the neck portion 5. The shape of the sleeve 2A is adjusted so as to be able to be combined with the taper shape corresponding to the standard of the neck portion 5 to be mounted, with respect to one head 8. Even though the illustrated sleeve 2A has a cylindrical form in which an end portion on a small diameter side is open, the sleeve 2A may have a cup-shaped form in which the end portion on the small diameter side is closed.

The sleeve 2A of the present embodiment has an inner circumferential portion 13 which makes contact with the outer circumferential surface 4 of the neck portion 5, and an outer circumferential portion 16 which makes contact with the inner circumferential surface 10 of the head 8. A second length L2 of the outer circumferential portion 16 is formed to be shorter compared to the overall length (equivalent to the overall length of the sleeve) L1 of the inner circumferential portion 13 when seen in the insertion direction A. In other words, an edge of the outer circumferential portion 16 on the large diameter side is set to be at a position farther away than an end surface 15 of an opening portion 14 in the insertion direction A by the distance ΔL1. Therefore, a thin inner circumferential section 17 having a third length L3 (= L1 - L2) is formed in the inner circumferential portion 13 based on a difference in length with respect to the outer circumferential portion 16. In this manner, when the sleeve 2A is seen in the insertion direction A, a contact position of the inner circumferential portion 13 with respect to the outer circumferential surface 4 of the neck portion 5 is different from a contact position of the outer circumferential portion 16 with respect to the inner circumferential surface 10 of the head 8.

It is desirable that the sleeve 2A does not protrude from the concavity 7 of the head 8. In a case of causing the sleeve 2A to protrude, it is desirable to set the sleeve 2A so as not to stick out of a virtual spherical surface including an outer surface of the head 8.

The inner circumferential portion 13 has the inner circumferential section 17 which is longer than the outer circumferential portion 16 by the third length L3, and thus, it is possible to effectively make contact with the outer circumferential surface 4 of the neck portion 5 and to ensure a length of fit L4 which is necessary with respect to the neck portion 5. In accordance with a thickness T of the sleeve 2A, it is possible to adjust the length of fit L4 and a neck offset (a distance from the center of the head 8 to a tip of the neck portion 5) with respect to the neck portion 5 of the head 8. In other words, if the thickness T of the sleeve 2A is increased, the inner diameter contracts, and thus, the length of fit L4 is shortened and the neck offset increases. In contrast, if the thickness T of the sleeve 2A is decreased, the inner diameter expands, and thus, the length of fit L4 is lengthened and the neck offset decreases. Even though there is no particular limitation for the thickness of the sleeve 2A, it is desirable to be practically in a range of 0.5 to 5.0 mm.

In this manner, since the length of fit L4 of the inner circumferential portion 13 and the outer circumferential surface 4 can be changed and a position of the head 8 with respect to the neck portion 5 can be adjusted by changing the thickness T of the sleeve 2A, it is possible to set different offsets in the same head 8 and stem 6 by preparing the sleeves 2A respectively having different thicknesses T.

Regarding a difference of the standards related to the taper shape of the neck portion 5, it is possible to use the same or a small number of types of the head 8 and the stem 6 by causing the sleeve 2A to be inserted. In other words, even though the stem 6 has a different standard, by causing the shape of the sleeve 2A disposed between the head 8 and the neck portion 5 to correspond thereto, it is possible to cause the head 8 and the neck portion 5 to be compatible with each other and to adjust the length of fit L4. In this manner, in accordance with the sleeve 2A to be inserted therebetween, it is possible to achieve a stable fitting state of the head 8 and the neck portion 5.

The outer circumferential portion 16 makes contact with the inner circumferential surface 10 of the head 8 from a position retracted further than the end surface 15 of the opening portion 14 by the length ΔL1, that is, from an intermediate portion of the concavity 7 in the insertion direction A, throughout the second length L2 in the insertion direction A. As the contact region of the outer circumferential portion 16 is retracted from the end surface 15, a region in which the head 8 makes contact with the outer circumferential portion 16 of the sleeve 2A is changed to a region having a relatively large cross-sectional area, avoiding a region of which the thickness is relatively thin in the range of the length ΔL1 from the end surface 15. Accordingly, it is possible to move a stress concentration portion of the head 8 to a position having the large cross-sectional area which is advantageous for strength, and to reduce the generated maximum principal stress.

The artificial hip joint 1A according to the present embodiment exhibits the effects described below. Since the region at which the inner circumferential surface 10 of the head 8 and the outer circumferential portion 16 of the sleeve 2A make contact with each other is changed to a region having a great thickness B and high strength, the place of stress concentration moves to this region, and thus, it is possible to prevent strength degradation and a fracture of the head 8 from occurring. Since the length of fit L4 of the outer circumferential surface 4 of the neck portion 5 of the stem 6 and the inner circumferential portion 13 of the sleeve 2A which is in contact therewith is caused to have a sufficient length, it is possible to ensure fitting strength with respect to the neck portion 5 of the head 8. Accordingly, it is possible to realize miniaturization of the head 8 by lowering an overall height H of the head 8 to the extent that the minimal necessary sliding area of the head can be obtained, and to exhibit excellent fixing strength by ensuring the sufficiently great length of fit L4 with respect to the overall height H of the head 8. The inner circumferential section 17 which is longer than the outer circumferential portion 16 by the third length L3 is provided in the inner circumferential portion 13 of the sleeve 2A, and thus, the inner circumferential section 17 becomes a guide when the head 8 is mounted on the neck portion 5. Therefore, it is possible to sufficiently lower the possibility of being fit obliquely with respect to the neck portion 5.

### (Analysis Example 1)

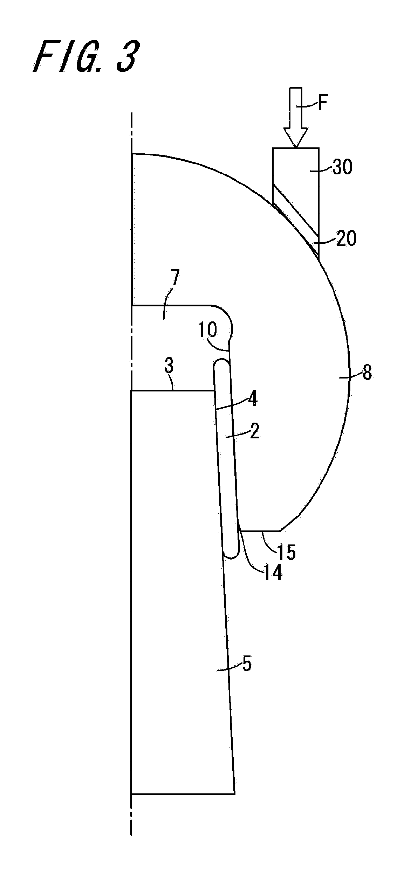

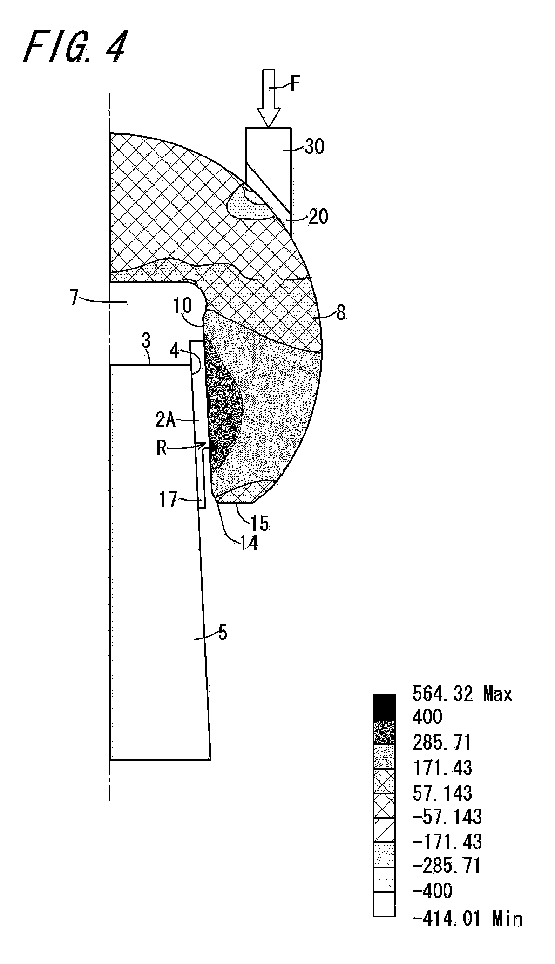

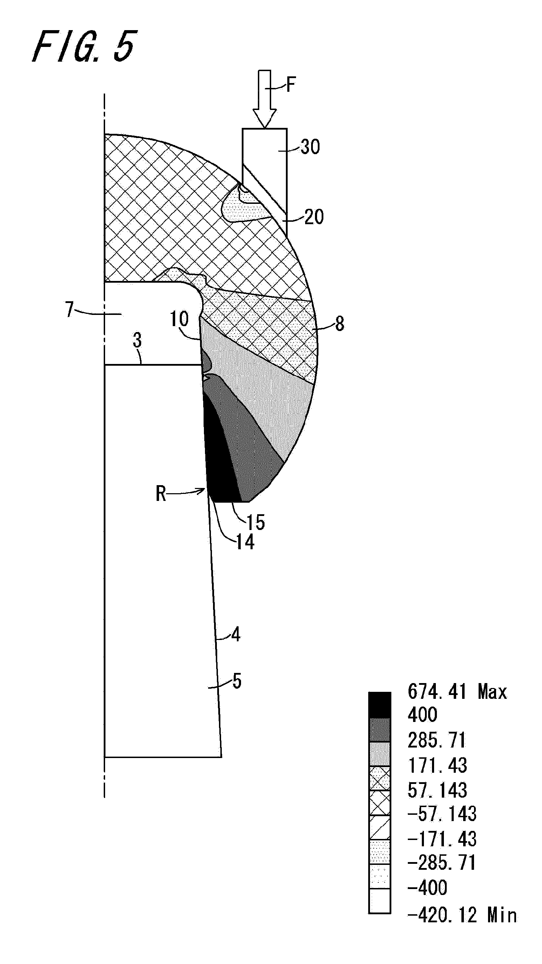

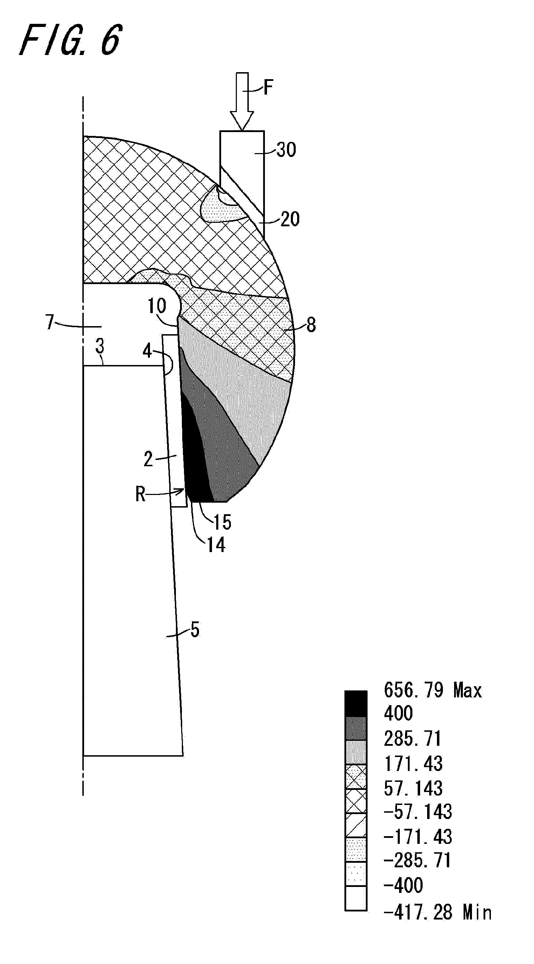

FIG. 3 is a diagram illustrating a stress analysis model of an artificial hip joint. FIG. 4 is a stress distribution diagram showing an analysis result of Example 1 simulating the artificial hip joint 1A illustrated in FIG. 1. FIG. 5 is a stress distribution diagram showing an analysis result of Comparison Example 1. FIG. 6 is a stress distribution diagram showing an analysis result of Comparison Example 2. In each diagram, the same reference numerals are assigned to portions corresponding to those of the artificial hip joint 1A in FIG. 1.

In the artificial hip joint 1A according to Embodiment 1, the inventors have carried out a stress analysis by using a finite element method (abbreviated to FEM) in order to identify the stress generated when an external force is applied to the head 8. As an analysis method, the stress analysis model illustrated in FIG. 3 is used. As an analysis target, the analysis model simulating the artificial hip joint 1A in FIG. 1 is "Example 1", the analysis model simulating the artificial hip joint without the sleeve is "Comparison Example 1", and the analysis model simulating the artificial hip joint having the simple conical sleeve having the regular thickness which is similar to the conventional technology is "Comparison Example 2".

In the stress analysis model of FIG. 3, the material of a member corresponding to the head 8 is alumina, the material of a member corresponding to the neck portion 5 of the stem is a cobalt-chromium-molybdenum (CCM) alloy, and the material of a member corresponding to the sleeves 2 and 2A is Ti-6Al-4V. Among Example 1, Comparison Example 1 and Comparison Example 2, the design of the head 8 is unified. The design of the neck portion 5 is the same in Example 1 and Comparison Example 2. However, in the design of the neck portion 5 in Comparison Example 1, only a portion corresponding to the thickness of the sleeve is caused to be thicker than that of the neck portion 5 in Example 1 and Comparison Example 2.

Then, there were calculated stress distribution generated in the head 8 when a load F of 46 kN is applied to the head 8 by using an iron pressing jig 30 having a copper ring 20 mounted on a tip thereof, and a maximum principal stress value. The load 46 kN used in the analysis is the criteria of average strength of the head 8 in accordance with the guidance of the Food and Drug Administration (abbreviated to FDA). Software used in the FEM analysis is general-purpose analysis software "ANSYS Workbench ver.13". Setting values for FEM analysis conditions are shown in Table 1. In Table 1, respectively, alumina corresponds to the head, CCM corresponds to the neck portion, Ti-6Al-4V corresponds to the sleeve, Fe corresponds to the pressing jig, and Cu corresponds to the copper ring.

**[Table 1]**

| Material | Friction Coefficient | Young's Modulus [GPa] |
|---|---|---|
| Alumina | 0.3 | 400 |
| CCM | 0.3 | 213 |
| Ti-6Al-4V | 0.3 | 110 |
| Fe | 0.3 | 200 |
| Cu | 0.3 | 120 |

### (FEM Analysis Result)

In Example 1 (Embodiment 1), Comparison Example 1 (no sleeve), and Comparison Example 2 (with the conical sleeve), stress distribution generated when the load F of 46 kN is applied to the head 8 by using the pressing jig 30 is illustrated in each of FIGS. 4, 5 and 6. In each diagram, a position generating the maximum principal stress is indicated by R. A maximum principal stress value (unit: MPa) obtained through the analysis is shown in Table 2.

**[Table 2]**

| | Shape | Maximum Principal Stress [MPa] |
|---|---|---|
| Example 1 | Embodiment 1 (FIG. 4) | 564.3 |
| Comparison Example 1 | No sleeve (FIG. 5) | 674.4 |
| Comparison Example 2 | Simple conical (FIG. 6) | 656.8 |

As seen from the analysis result, it has been confirmed that the maximum principal stress value generated in the head 8 is the lowest in Example 1 using the sleeve 2A according to Embodiment 1. In Example 1, the contact point of the sleeve 2A and the head 8 is set to a place having a large cross-sectional area, avoiding the thin opening portion 14 and the vicinity thereof, and thus, as illustrated in FIG. 4, the position R generating the maximum principal stress moves deep inside the concavity 7 in the insertion direction A. As a result, compared to Comparison Example 1 and Comparison Example 2, it is considered that the maximum principal stress value decreases in Example 1. In accordance with this, it is considered that fracture strength increases. Moreover, since the interface increases by causing the sleeve 2A to be inserted compared to a case of not having the sleeve 2A, sliding is generated between the head 8 and the neck portion 5, thereby being actuated to decrease stress generated in the head 8, by the sliding. Therefore, it is considered that an effect of decreasing stress generated in the head 8 is exhibited in cooperation with this action.

In Comparison Example 1 illustrated in FIG. 5, the head 8 and the neck portion 5 make direct contact with each other. Therefore, as illustrated in FIG. 5, stress is concentrated in the vicinity of the opening portion 14 which is the thinnest portion in the head 8. In addition, the value of the maximum principal stress is also much greater than that in Example 1. Furthermore, since stress is concentrated at the thin region in the vicinity of the opening portion 14, the maximum principal stress value increases. As a result, it is considered that fracture strength is lowered.

In Comparison Example 2 illustrated in FIG. 6, stress is concentrated in the vicinity of the thinnest opening portion 14 in the head 8. In addition, the value of the maximum principal stress is also much greater than that in Example 1. However, in Comparison Example 2, interfaces increase by causing the sleeve 2 to be inserted between the head 8 and the neck portion 5, thereby achieving a decrease of stress due to the sliding. In accordance with such a stress decreasing action achieved by the sleeve 2, compared to Comparison Example 1 having no sleeve, it is considered that the maximum principal stress value slightly decreases. As a result, compared to Comparison Example 1, in Comparison Example 2, it is considered that fracture strength slightly increases.

According to the analysis result described above, compared to a case of using no sleeve (Comparison Example 1) or a case of using the conventional simple conical sleeve 2 (Comparison Example 2), it has been confirmed that the maximum principal stress value decreases by using the sleeve 2A in Example 1. As a result, it is considered that fracture strength of the head 8 is improved.

### (Embodiment 2)

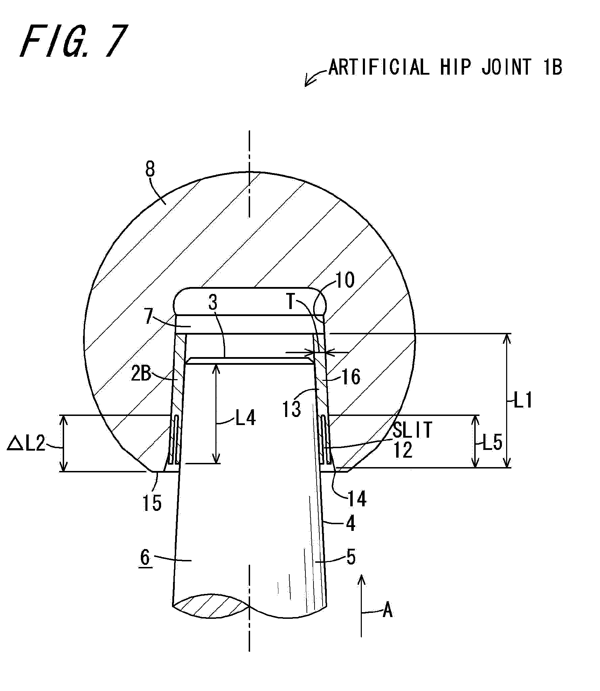

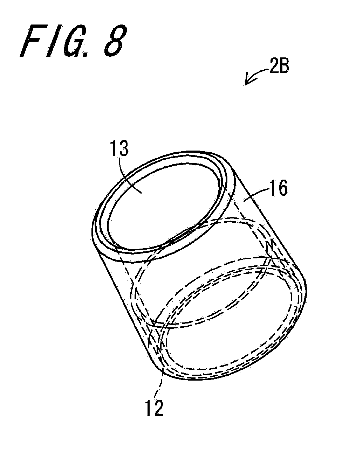

FIG. 7 is a cross-sectional view illustrating an artificial hip joint 1B according to Embodiment 2 of the invention. FIG. 8 is a perspective view of a sleeve 2B which is used in the artificial hip joint 1B. The same reference numerals are assigned to portions corresponding to those in Embodiment 1 described above.

The artificial hip joint 1B of the present embodiment, in common with Embodiment 1, includes the stem 6, the head 8, and the sleeve 2B. The stem 6 has the neck portion 5 which has the truncated conical outer circumferential surface 4 decreased in diameter toward the tip thereof. The head 8 is provided with the concavity 7 in which the neck portion 5 is inserted, and the head 8 has the inner surface defining the concavity 7 which inner surface includes the truncated conical inner circumferential surface 10 decreased in diameter toward the insertion direction of inserting the neck portion 5. The sleeve 2B is inserted between the outer circumferential surface 4 of the neck portion 5 inserted into the concavity 7, and the inner circumferential surface 10 of the head 8.

The sleeve 2B has the inner circumferential portion 13 which makes contact with the outer circumferential surface 4 of the neck portion 5, and the outer circumferential portion 16 which makes contact with the inner circumferential surface 10 of the head 8. A slit 12 which has a substantially cylindrical shape in a mounted state and extends in the insertion direction A from one end portion arranged on the opening portion side 14 of the sleeve 2B is formed between the inner circumferential portion 13 and the outer circumferential portion 16.

As the slit 12 is formed in the sleeve 2B, the vicinity of the opening portion 14 of the head 8 is deformed when a load is applied to the head 8. Therefore, in accordance with this deformation, the outer circumferential portion 16 of the sleeve 2B which makes contact with the inner circumferential surface 10 of the head 8 can be warped. As a result, it is possible to prevent stress from being concentrated at a relatively thin portion, within a range of the uniform length ΔL2 from the end surface 15 of the head 8. Thus, it is possible to improve fracture strength of the head 8.

A forming length L5 of the slit 12 in the sleeve 2B is set such that the outer circumferential portion 16 can be warped following deformation which occurs when the head 8 receives a load, thereby allowing the stress concentration portion to move to a place where the thickness of the head 8 is relatively great.

In the present embodiment, similar to the conventional simple conical sleeve 2, the lengths of fit on the inner and outer surfaces of the sleeve 2B are set to be equivalent to each other and to have sufficient lengths. Therefore, a risk of obliquely fitting the neck portion 5 can be decreased similar to the conventional simple conical sleeve 2.

### (Analysis Example 2)

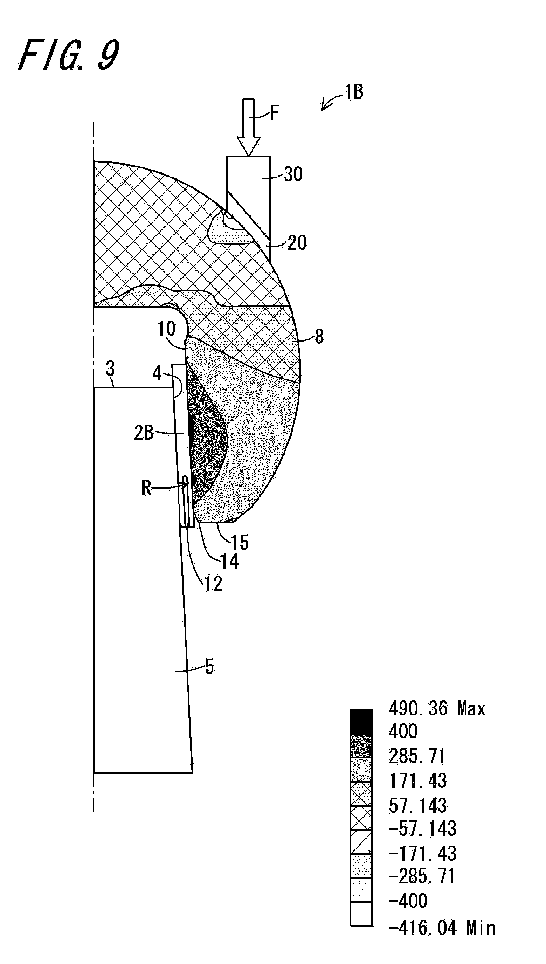

An analysis has been carried out similar to that in Embodiment 1 regarding stress generated when an external force is applied to the head 8 of the artificial hip joint 1B in Embodiment 2. FIG. 9 is a stress distribution diagram showing an analysis result of a model simulating the artificial hip joint 1B in the present embodiment.

The analysis method complies with that in Analysis Example 1. In other words, the stress analysis model illustrated in FIG. 3 is used. As the analysis target, the analysis model simulating the artificial hip joint 1B in FIG. 7 is adopted to be "Example 2", thereby analyzing stress generated when the load F of 46 kN is applied to the head 8, through the FEM analysis. Software used in the FEM analysis is general-purpose analysis software "ANSYS Workbench ver.13" similar to that in Analysis Example 1. The setting values for the FEM analysis conditions are in common with those in Analysis Example 1.

### (FEM Analysis Result)

The analysis result is shown in FIG. 9 and Table 3. "Comparison Example 1" and "Comparison Example 2" in Table 3 are in common with those in Analysis Example 1.

**[Table 3]**

| | Shape | Maximum Principal Stress [MPa] |
|---|---|---|
| Example 2 | Embodiment 2 (FIG. 9) | 490.4 |
| Comparison Example 1 | No sleeve (FIG. 5) | 674.4 |
| Comparison Example 2 | Simple conical (FIG. 6) | 656.8 |

As seen from the analysis result, it has been confirmed that the maximum principal stress value generated in the head 8 decreases by using the sleeve 2B of Embodiment 2. In Example 2, since the sleeve 2B has the slit 12, as illustrated in FIG. 9, the position R generating the maximum principal stress moves from the opening portion 14 of the concavity 7 to deep inside thereof. As a result, the maximum principal stress value decreases. In addition, since interfaces increase by causing the sleeve 2B to be inserted, sliding is generated among the head 8, the sleeve 2B and the neck portion 5, thereby being actuated to decrease stress generated in the head 8, by the sliding. Therefore, it is considered that an effect of decreasing stress generated in the head 8 is exhibited in cooperation with this action.

To summarize the above descriptions, compared to a case of using no sleeve (Comparison Example 1) or a case of using the conventional simple conical sleeve 2 (Comparison Example 2), it is possible to improve fracture strength of the head 8 by using the sleeve 2B in the present embodiment.

### (Embodiment 3)

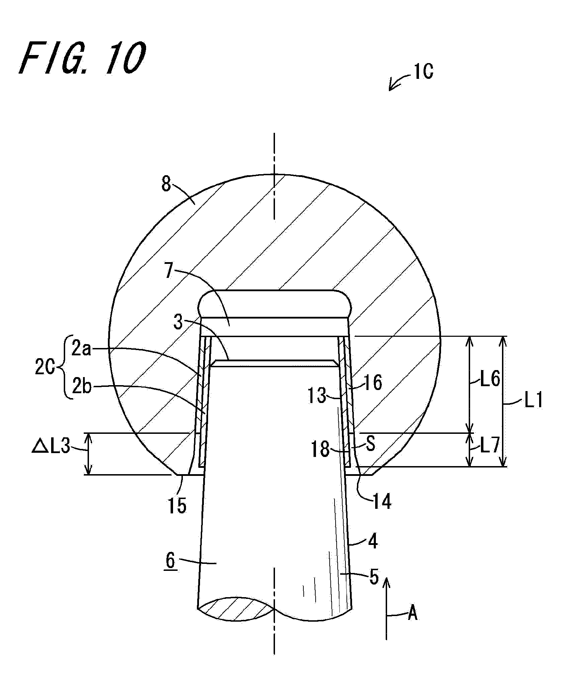

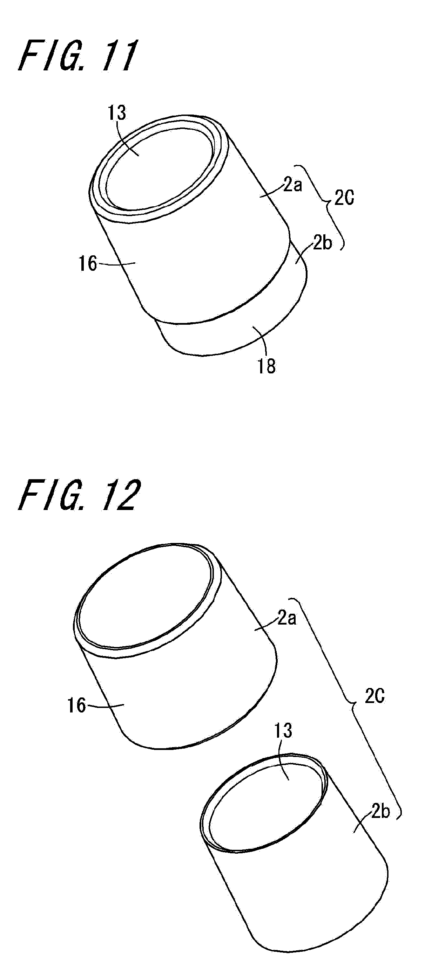

FIG. 10 is a cross-sectional view illustrating an artificial hip joint 1C according to Embodiment 3 of the invention. FIG. 11 is a perspective view of a sleeve 2C which is used in the artificial hip joint 1C. FIG. 12 is an exploded perspective view of the sleeve 2C which is used in the artificial hip joint 1C. The same reference numerals are assigned to portions corresponding to those in Embodiment 1.

The artificial hip joint 1C of the present embodiment includes the stem 6, the head 8, and the sleeve 2C. The stem 6 has the neck portion 5 which has the truncated conical outer circumferential surface 4 decreased in diameter toward a tip thereof. The head 8 is provided with the concavity 7 in which the neck portion 5 is inserted, and the head 8 has the inner surface defining the concavity 7 which inner surface includes the truncated conical inner circumferential surface 10 decreased in diameter toward the insertion direction of inserting the neck portion 5. The sleeve 2C is inserted between the outer circumferential surface 4 of the neck portion 5 inserted into the concavity 7, and the inner circumferential surface 10 of the head 8. The sleeve 2C has a multi-layer structure in which a plurality of sleeve sections having substantially the same shape are stacked. In the present embodiment, the sleeve 2C has a two-layer structure.

As illustrated in FIGS. 11 and 12, the sleeve 2C of the present embodiment has a hollow truncated conical outer cylinder section 2a and a hollow truncated conical inner cylinder section 2b which is fit inside the outer cylinder section 2a and of which the length in an axial direction is formed to be longer than the outer cylinder section 2a. In the sleeve 2C, the outer circumferential surface of the outer cylinder section 2a constitutes the outer circumferential portion 16 which makes contact with the inner circumferential surface 10 of the head 8, and the inner circumferential surface of the inner cylinder section 2b constitutes the inner circumferential portion 13 which makes contact with the outer circumferential surface 4 of the neck portion 5. In the present embodiment, when the outer cylinder section 2a is stacked on the inner cylinder section 2b, end surfaces on small diameter sides thereof are set to be flush with each other.

In the sleeve 2C, an overall length L6 of the outer cylinder section 2a is formed to be shorter compared to the overall length (equivalent to the overall length of the sleeve) L1 of the inner cylinder section 2b when seen in the insertion direction A. Therefore, when the inner cylinder section 2b and the outer cylinder section 2a are stacked, the inner cylinder section 2b protrudes from one end of the outer cylinder section 2a by a length of a difference L7 (= L1 - L6) of the lengths therebetween. A space S is formed between a protrusion section 18 and the inner circumferential surface 10 of the head 8.

The space S is formed in the vicinity of the opening portion 14 of the sleeve 2C so as to provide a region which does not make contact with the inner circumferential surface 10 of the head 8. Thus, when a load is applied to the head 8, it is possible to move the stress concentration portion to a region having a great thickness deep inside the concavity 7 and being away from the end surface 15 of the head 8 by a uniform length ΔL3. As a result, in the sleeve 2C, it is possible to prevent stress from being concentrated at a relatively thin portion in the vicinity of the opening portion 14. Therefore, it is possible to improve fracture strength of the head 8.

Moreover, the sleeve 2C of the present embodiment has a structure in which the outer cylinder section 2a and the inner cylinder section 2b having substantially the same shape are stacked in a double layer. Therefore, compared to a case where the sleeve is in a single body, sliding surfaces between the outer cylinder section 2a and the inner cylinder section 2b increase, and thus, it is possible to decrease stress generated in the head 8.

The sleeve 2C of the present embodiment is composed of the combination of the simple conical outer cylinder section 2a and inner cylinder section 2b, thereby being easily processed and being excellent in productivity. Furthermore, due to the multi-layer structure, the thickness adjustment is easy. The outer cylinder section 2a and the inner cylinder section 2b are not necessarily tapered in the same manner. The sleeve 2C can have a multi-layer structure including equal to or more than three layers.

### (Analysis Example 3)

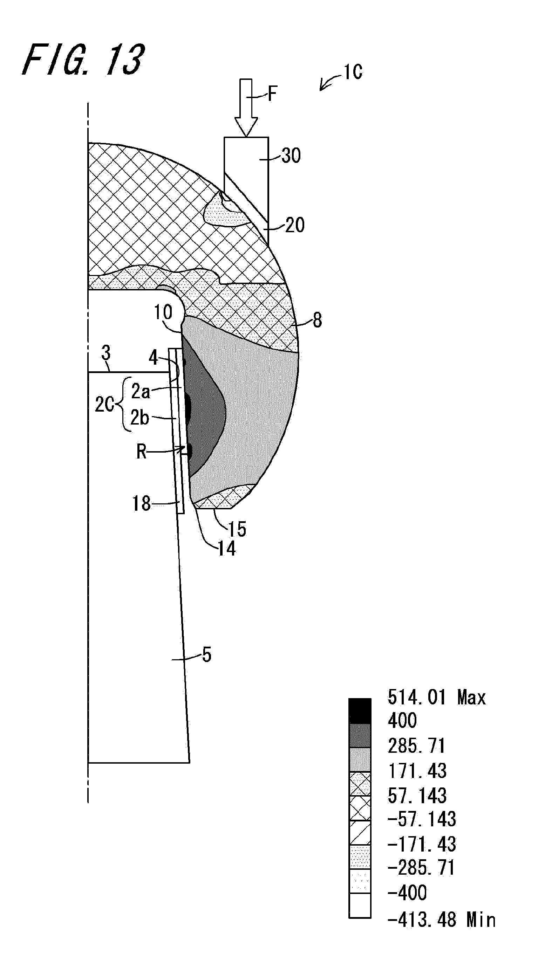

An analysis has been carried out similar to that in Embodiment 1 regarding stress generated when an external force is applied to the head 8 of the artificial hip joint 1C in Embodiment 3. FIG. 13 is a stress distribution diagram showing an analysis result of a model simulating the artificial hip joint 1C in the present embodiment. The analysis method complies with that in Analysis Example 1. In other words, the stress analysis model illustrated in FIG. 3 is used. As the analysis target, the analysis model simulating the artificial hip joint 1C in FIG. 10 is adopted to be "Example 3", thereby analyzing stress generated when the load F of 46 kN is applied to the head 8, through the FEM analysis. Software used in the FEM analysis is general-purpose analysis software "ANSYS Workbench ver.13" similar to that in Analysis Example 1. The setting values for the FEM analysis conditions are in common with those in Analysis Example 1.

### (FEM Analysis Result)

The analysis result is shown in FIG. 13 and Table 4. "Comparison Example 1" and "Comparison Example 2" in Table 4 are in common with those in Analysis Example 1.

**[Table 4]**

| | Shape | Maximum Principal Stress [MPa] |
|---|---|---|
| Example 3 | Embodiment 3 (FIG. 13) | 514.0 |
| Comparison Example 1 | No sleeve (FIG. 5) | 674.4 |
| Comparison Example 2 | Simple conical (FIG. 6) | 656.8 |

As seen from the analysis result, it has been confirmed that the maximum principal stress value generated in the head 8 decreases by using the sleeve 2C of Embodiment 3. In Example 3, a position where the outer cylinder section 2a of the sleeve 2C makes contact with the inner circumferential surface 10 of the head 8 is separated from the opening portion 14. Therefore, as illustrated in FIG. 13, the position R generating the maximum principal stress moves from the opening portion 14 of the concavity 7 to deep inside thereof. As a result, the maximum principal stress value decreases. In addition, since interfaces increase by causing the sleeve 2C having the two-layer structure to be inserted, sliding is generated between the head 8 and the neck portion 5, thereby being actuated to decrease stress generated in the head 8, by the sliding. Therefore, it is considered that an effect of decreasing stress generated in the head 8 is exhibited in cooperation with this action.

To summarize the above descriptions, in the artificial hip joint 1C of the present embodiment, since the stress concentration portion moves to a region having great strength, it is possible to decrease the maximum principal stress value generated in the head 8 and to improve fracture strength of the head 8. In addition, by using the sleeve 2C having the multi-layer structure, the interfaces increase and sliding is generated between the outer cylinder section 2a and the inner cylinder section 2b, and thus, this also allows an effect of decreasing stress generated in the head 8 to be achieved.

### (Embodiment 4)

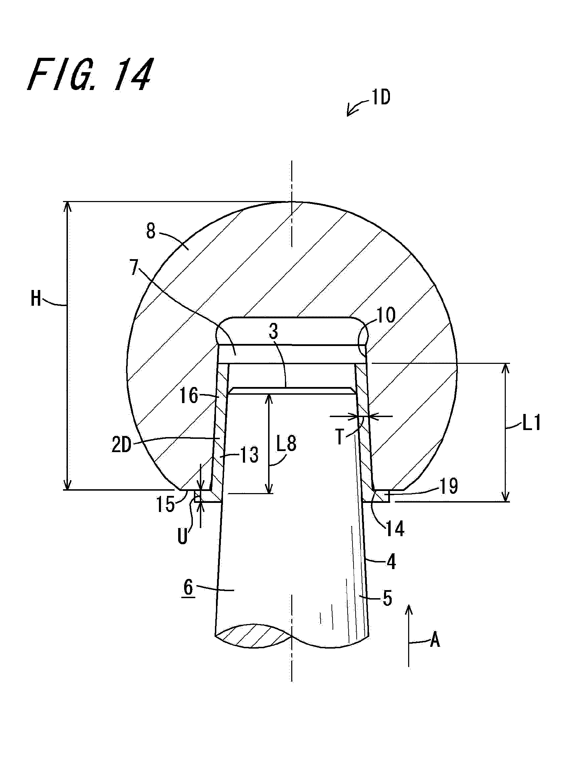

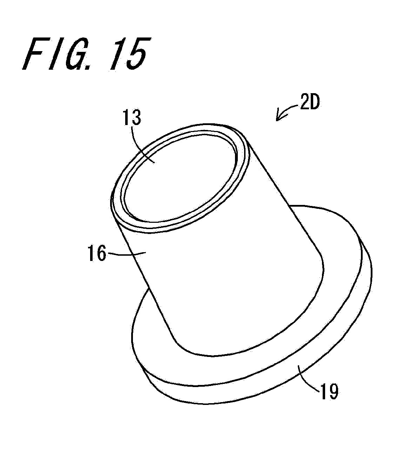

FIG. 14 is a cross-sectional view illustrating an artificial hip joint 1D according to Embodiment 4 of the invention. FIG. 15 is a perspective view of a sleeve 2D which is used in the artificial hip joint 1D. The same reference numerals are assigned to portions corresponding to those in the above-described embodiments.

The artificial hip joint 1D of the present embodiment, in common with Embodiment 1, includes the stem 6, the head 8, and the sleeve 2D. The stem 6 has the neck portion 5 which has the truncated conical outer circumferential surface 4 decreased in diameter toward the tip thereof. The head 8 is provided with the concavity 7 in which the neck portion 5 is inserted, and the head 8 has the inner surface defining the concavity 7 which inner surface includes the truncated conical inner circumferential surface 10 decreased in diameter toward the insertion direction of inserting the neck portion 5. The sleeve 2D is inserted between the outer circumferential surface 4 of the neck portion 5 inserted into the concavity 7, and the inner circumferential surface 10 of the head 8.

the sleeve 2D of the present embodiment has the inner circumferential portion 13 which makes contact with the outer circumferential surface 4 of the neck portion 5, the outer circumferential portion 16 which makes contact with the inner circumferential surface 10 of the concavity 7, and a flange portion 19 which is widened toward the opening portion 14 side. Then, the flange portion 19 formed at the end portion on the opening portion 14 side is configured to be in surface contact with the end surface 15 which is a peripheral surface of the opening portion 14 of the head 8. In the present embodiment, the end surface 15 of the head 8 is set at a place having a relatively great thickness.

It is desirable to set the radius of the flange portion 19 and a thickness U seen in the insertion direction A so as to prevent the flange portion 19 from sticking out of the virtual spherical surface including the outer surface of the head 8. Therefore, it is desirable that, when increasing the radius of the flange portion 19, the thickness U is formed to be thin, and when increasing the thickness U, the radius of the flange portion 19 is formed to be small.

In the sleeve 2D of the present embodiment, differently from Embodiments 1 to 3, there is no need to ensure a region for forming the space S or the slit 12 in the vicinity of the opening portion 14 of the head 8. In other words, since the outer circumferential portion 16 of the sleeve 2D makes contact with the inner circumferential surface 10 of the head 8 from the position of the opening portion 14, even though the overall height H of the head 8 is decreased, it is possible to ensure the sufficient length of fit. Accordingly, since the head 8 can be decreased in size to the extent that the minimal necessary caput sliding area can be obtained, it is possible to provide a small head 8. As the head 8 is decreased in size, the sliding area also decreases, and thus, it is easy to perform polishing of the surface of the head 8. In the sleeve 2D, in order to decrease the maximum principal stress value, the space S or the slit 12 may be formed in the vicinity of the opening portion 14 of the head 8.

Moreover, in the sleeve 2D, since the length of the inner circumferential portion 13 when seen in the insertion direction A is a length including the flange portion 19, a length of fit L8 with the neck portion 5 can be elongated with respect to the overall height H of the head 8. Accordingly, when the neck portion 5 is fit inside the sleeve 2D so as to fix the head 8 to the stem 6, it is possible to lower the possibility that the neck portion 5 fits obliquely, and to prevent fracture strength of the head 8 from being degraded.

In the artificial hip joint 1D according to the present embodiment, when a load acts on the head 8, the load also acts on the sleeve 2D through the head 8. Since the sleeve 2D receives the load at the outer circumferential portion 16 and the flange portion 19, stress generated in the head 8 is dispersed in a direction from the opening portion 14 along the outer circumferential portion 16 and a direction along the flange portion 19. As a result, the value of the maximum principal stress decreases. Moreover, in the present embodiment, since the end surface 15 which makes contact with the flange portion 19 is set to a place having a great thickness, it is possible to improve fracture strength.

### (Analysis Example 4)

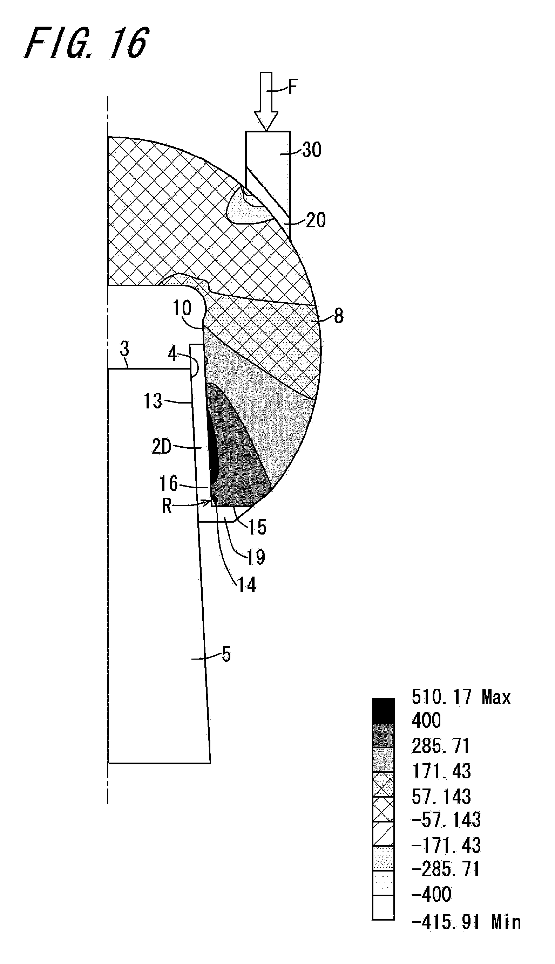

An analysis has been carried out similar to that in Embodiment 1 regarding stress generated when an external force is applied to the head 8 of the artificial hip joint 1D in Embodiment 4. FIG. 16 is a stress distribution diagram showing an analysis result of a model simulating the artificial hip joint 1D in the present embodiment. The analysis method complies with that in Analysis Example 1. In other words, the stress analysis model illustrated in FIG. 3 is used. As the analysis target, the analysis model simulating the artificial hip joint 1D in FIG. 14 is adopted to be "Example 4", thereby analyzing stress generated when the load F of 46 kN is applied to the head 8, through the FEM analysis. Software used in the FEM analysis is general-purpose analysis software "ANSYS Workbench ver.13" similar to that in Analysis Example 1. The setting values for the FEM analysis conditions are in common with those in Analysis Example 1.

### (FEM Analysis Result)

The analysis result is shown in FIG. 16 and Table 5. "Comparison Example 1" and "Comparison Example 2" in Table 5 are in common with those in Analysis Example 1.

**[Table 5]**

| | Shape | Maximum Principal Stress [MPa] |
|---|---|---|
| Example 4 | Embodiment 4 (FIG. 16) | 510.2 |
| Comparison Example 1 | No sleeve (FIG. 5) | 674.4 |
| Comparison Example 2 | Simple conical (FIG. 6) | 656.8 |

As seen from the analysis result, it has been confirmed that the maximum principal stress value generated in the head 8 decreases by using the sleeve 2D of Embodiment 4. In Example 4, as illustrated in FIG. 16, even though the position R generating the maximum principal stress is in the vicinity of the opening portion 14, the maximum principal stress value is sufficiently small compared to Comparison Examples 1 and 2. It is considered that since the flange portion 19 which makes contact with the end surface 15 of the head 8 is formed in the sleeve 2D, when the load F is applied to the head 8, stress generated in the vicinity of the opening portion 14 of the head 8 is dispersed not only in the direction of the outer circumferential portion 16 but also in the direction of the flange portion 19.

According to each of the above-described embodiments according to the invention, an excellent effect is exhibited as follows. By adjusting the shapes of the sleeves 2A to 2D in accordance with the taper shapes in various standards of the neck portion 5 of the stem 6, it is possible to select the taper shape to be mounted out of the multiple types with respect to one head 8. By adjusting the thicknesses T of the sleeves 2A to 2D, it is possible to change the neck offset with one head 8.

Each of the artificial hip joints 1A to 1D according to the invention is configured to include the head 8 made of ceramics, and the stem 6 and the sleeves 2A to 2D which are made of metal. Therefore, combinations of various types of the heads 8 and various types of the stems 6 are possible according to selection of the sleeves 2A to 2D, and thus, it is possible to provide various types of artificial hip joints having various sizes and materials.

In addition, since the length of taper-fit in which the outer circumferential portions 16 of the sleeves 2A to 2D make contact with the inner circumferential surface 10 of the head 8, and the length of taper-fit in which the inner circumferential portion 13 makes contact with the outer circumferential surface 4 of the neck portion 5 of the stem 6 can respectively ensure the sufficient lengths, it is possible to lower the possibility that the neck portion 5 fits obliquely. Moreover, since it is possible to move the stress concentration portion of the head 8 to the position which is advantageous for strength or to disperse stress, it is possible to decrease the maximum principal stress value of the head 8, and to improve fracture strength of the head 8.

In the illustrated embodiments, each taper angle between the outer circumferential portions 16 of the sleeves 2A to 2D and the inner circumferential surface 10 of the head 8 is caused to be the same so as to make surface contact therewith. However, in order to decrease the maximum principal stress value of the head 8 further and improve fracture strength of the head 8 further, the taper angles of the outer circumferential portions 16 of the sleeves 2A to 2D are caused to be smaller than the taper angle of the inner circumferential surface 10 of the head 8, and thus, the sleeves 2A to 2D mounted on the neck portion 5 may be brought into contact with the inner circumferential surface 10 of the concavity 7 at the position deeper than the concavity 7 of the head 8 in the insertion direction of inserting the neck portion 5, that is, a so-called "deep" fitting structure.

In addition, in order to improve fracture strength of the head 8 further, the taper angles of the inner circumferential portions 13 of the sleeves 2A to 2D are increased to be greater than the taper angle of the outer circumferential surface 4 of the neck portion 5, and thus, the tip 3 of the neck portion 5 may be brought into contact with the inner circumferential portion 13 at the position inside the sleeves 2A to 2D, that is, the so-called "deep" fitting structure.

The invention may be embodied in other specific forms without departing from the spirit or essential characteristics thereof. The present embodiments are therefore to be considered in all respects as illustrative and not restrictive, the scope of the invention being indicated by the appended claims rather than by the foregoing description and all changes which come within the meaning and the range of equivalency of the claims are therefore intended to be embraced therein.

### Reference Signs List

- 1A to 1D:: Artificial hip joint
- 2A to 2D:: Sleeve
- 3:: Tip
- 4:: Outer circumferential surface
- 5:: Neck portion
- 6:: Stem
- 7:: Concavity
- 8:: Head
- 9:: Inner surface
- 10:: Inner circumferential surface
- 13:: Inner circumferential portion
- 14:: Opening portion
- 15:: End surface
- 16:: Outer circumferential portion
- 17:: Inner circumferential section
- 18:: Protrusion section
- 19:: Flange portion

## Claims

1. An artificial hip joint, comprising:
a stem having a neck portion which has a truncated conical outer circumferential surface decreased in diameter toward a tip thereof;
a head provided with a concavity in which the neck portion is inserted, an inner surface of the concavity of the head including a truncated conical inner circumferential surface decreased in diameter toward an insertion direction of inserting the neck portion; and
a sleeve inserted between an outer circumferential surface of the neck portion inserted into the concavity and an inner circumferential surface of the head,
the sleeve having an inner circumferential portion which makes contact with the outer circumferential surface of the neck portion, and an outer circumferential portion which makes contact with the inner circumferential surface of the head, a starting end of the outer circumferential portion being located at a position retracting from an opening portion of the concavity in the insertion direction.

2. An artificial hip joint, comprising:
a stem having a neck portion which has a truncated conical outer circumferential surface decreased in diameter toward a tip thereof;
a head provided with a concavity in which the neck portion is inserted, an inner surface of the concavity of the head including a truncated conical inner circumferential surface decreased in diameter toward an insertion direction of inserting the neck portion; and
a sleeve inserted between an outer circumferential surface of the neck portion inserted into the concavity and an inner circumferential surface of the head,
the sleeve having an inner circumferential portion which makes contact with the outer circumferential surface of the neck portion, and an outer circumferential portion which makes contact with the inner circumferential surface of the head, the sleeve being provided with a slit which is formed between the inner circumferential portion and the outer circumferential portion so as to extend in the insertion direction from one end portion of the sleeve located on an opening portion side of the concavity.

3. An artificial hip joint, comprising:
a stem having a neck portion which has a truncated conical outer circumferential surface decreased in diameter toward a tip thereof;
a head provided with a concavity in which the neck portion is inserted, an inner surface of the concavity of the head including a truncated conical inner circumferential surface decreased in diameter toward an insertion direction of inserting the neck portion; and
a sleeve inserted between an outer circumferential surface of the neck portion inserted into the concavity and an inner circumferential surface of the head,
the sleeve having a multi-layer structure in which a plurality of sleeve sections are stacked.

4. An artificial hip joint, comprising:
a stem having a neck portion which has a truncated conical outer circumferential surface decreased in diameter toward a tip thereof;
a head provided with a concavity in which the neck portion is inserted, an inner surface of the concavity of the head including a truncated conical inner circumferential surface decreased in diameter toward an insertion direction of inserting the neck portion; and
a sleeve inserted between an outer circumferential surface of the neck portion inserted into the concavity and an inner circumferential surface of the head,
the sleeve having an inner circumferential portion which makes contact with the outer circumferential surface of the neck portion, an outer circumferential portion which makes contact with the inner circumferential surface of the concavity, and a flange portion which makes contact with a peripheral surface of an opening portion of the concavity.

5. The artificial hip joint according to any one of claims 1 to 4,
wherein the sleeve is made of Ti or a Ti alloy.
